# EUROPEAN PATENT SPECIFICATION

(11) **EP 3 902 508 B1**
(45) Date of publication and mention of the grant of the patent: **10.08.2022**
(21) Application number: 19838968.6
(22) Date of filing: 20.12.2019
(51) Int. Cl.: A61F 13/15, A61F 5/453, A61F 13/471

(54) **URINE ABSORBENT BAG AND RELATED METHOD OF MANUFACTURING**
URINABSORBIERENDER BEUTEL UND VERFAHREN ZUR HERSTELLUNG
POCHE À URINE ET PROCÉDÉ DE FABRICATION ASSOCIÉ

(30) Priority: 27.12.2018 IT 201800021124
(43) Date of publication of application: 03.11.2021
(73) Proprietor: Proger Healthcare Srl, 65123 Pescara (IT)
(72) Inventor: SGAMBATI, Umberto, 65123 Pescara (IT); D'ALCINI, Franco, 65123 Pescara (IT)
(74) Representative: IP Sextant s.r.l.
(86) International application number: PCT/IB2019/061177
(87) International publication number: WO 2020/136526

(56) References cited:
- EP-A1- 0 776 642
- EP-A2- 1 086 679
- WO-A1-2010/071517
- US-A- 4 590 931

## Description

The present invention concerns an urine absorbent bag, intended for the care and hygiene of male persons, in particular affected by light incontinence, e.g. senile incontinence, that is wearable in a simple way, discreet in terms of wearability, adaptable and reliable to protect and contain the penis, and that allows to absorb leakage of urine or other body fluids in a reliable and efficient way, also being simple and inexpensive to manufacture.

The present invention further concerns a method of manufacturing such urine absorbent bag.

As known, many male patients suffer from urinary incontinence.

To prevent such a male patient from getting dirty, staining clothes and giving off a bad smell, absorbent diapers are used in the prior art.

However, these absorbent diapers are rather bulky and uncomfortable, thus other sanitary articles have been developed in the prior art for male patients only suffering from light incontinence, such as for instance senile incontinence. Such prior art sanitary items are bags provided with an absorbent tissue, into which the penis can be inserted. Some examples of these bags are described in documents WO 2014/053417 A1 and EP 787472 A1.

However, even these bags suffer from some drawbacks, mainly due to the fact that they are not wearable in a simple, adaptable and reliable way and that they do not absorb urine leakage in a reliable and efficient way. Moreover, they are often not simple and inexpensive to manufacture.

It is an object of this invention, therefore, to provide an urine absorbent bag, in particular intended for male people suffering from da light incontinence, e.g. senile incontinence, that is wearable in a simple way, discreet in terms of wearability, adaptable and reliable, and that allows to absorb leakage of urine or other body fluids of the user in a reliable and efficient way.

It is a further object of this invention to provide such a urine absorbent bag that is also simple and inexpensive to manufacture.

It is specific subject-matter of the present invention an urine absorbent bag configured to house a penis of a user, having a planar multilayer structure folded in "C"-shape to form said absorbent bag having a longitudinal axis, wherein said absorbent bag has a top opening, configured to receive the penis of the user, that is defined by a top edge of the planar multilayer structure, wherein said absorbent bag further has a closed bottom defined by a bottom edge of the planar multilayer structure, whereby a first lateral portion of the planar multilayer structure is overlapping a central portion of the planar multilayer structure so that a first lateral edge thereof is placed below a second lateral portion of the planar multilayer structure, wherein the planar multilayer structure comprises an inner layer configured to come into contact with the penis of the user, an outer layer and an absorbent core interposed between the inner layer and the outer layer, wherein the absorbent core is configured to absorb and retain fluid, wherein an end portion of a second lateral edge of the planar multilayer structure, longitudinally extending from the bottom edge to a length not greater than 50% of the length of the second lateral edge, is permanently coupled onto the first lateral portion.

According to another aspect of the invention, the end portion of the second lateral edge of the planar multilayer structure can longitudinally extend from the bottom edge to a length not greater than 40% of the length of the second lateral edge.

According to another aspect of the invention, the inner layer or the outer layer may be provided with an adhesive label configured to adjustably couple the second lateral edge onto the first lateral portion, wherein the adhesive label is optionally repositionable and/or extensible.

According to an additional aspect of the invention, the absorbent core may comprise a liquid and fluid absorption area made of at least one absorbent powder, optionally a superabsorbent polymer powder or SAP, trapped between two films of cellulosic material, wherein an amount of said at least one absorbent powder in the absorption area is varying, optionally in a continuous and/or stepped way:
- along the longitudinal axis, whereby the absorption area is divided into two zones, optionally of equal extension in the plane of the planar multilayer structure, consisting in a zone with reduced absorption and in a storage zone, wherein the zone with reduced absorption is closer to the top edge with respect to the storage zone, wherein the amount of said at least one absorbent powder is higher in the storage zone with respect to the zone with reduced absorption; and/or
- along an axis orthogonal to the longitudinal axis.

According to another aspect of the invention, the amount of said at least one absorbent powder in the absorption area may be varying along the longitudinal axis and the amount of said at least one absorbent powder in the storage zone may be variable from 60% to 90%, optionally equal to at least 70%, of the amount of said at least one absorbent powder in the entire absorption area.

According to another aspect of the invention, the absorption area may be composed of at least three absorption sub-areas extending parallel to the longitudinal axis, wherein at least two longitudinal flow channels devoid of said at least one absorbent powder separate said at least three absorption sub-areas from each other.

According to an additional aspect of the invention, the planar multilayer structure may further comprise an intermediate distribution layer interposed between the inner layer and the absorbent core, wherein the intermediate distribution layer is configured to acquire and distribute body fluids towards the absorption area.

According to another aspect of the invention, the intermediate distribution layer may be made of a material composed of:
- natural fibres, optionally selected from the group comprising or consisting of celluloses, cellulose derivatives, viscose, bamboo, and combinations thereof, and/or
- synthetic polymeric fibres, optionally selected from the group comprising or consisting of polyethylene, polypropylene, polyester, two-component fibres, and combinations thereof, and/or
- combinations of said natural fibres and said synthetic polymeric fibres.

According to another aspect of the invention, the inner layer may be provided with:
- one or more, optionally two, first transfer pattern and/or embossing lines extending along a direction parallel to the longitudinal axis to a longitudinal length optionally not smaller than 60% of the longitudinal length of the absorption area, wherein said one or more first transfer pattern and/or embossing lines are configured to guide a body fluid towards the closed bottom, and/or
- one or more, optionally two, second barrier pattern and/or embossing lines extending along a direction transverse to the longitudinal axis, optionally in correspondence with transverse edges of the absorption area, optionally for a transverse length not smaller than 70% of the transverse length of the absorption area, wherein said one or more second barrier pattern and/or embossing lines are configured to operate as protection barrier to prevent leakage of body fluids stored in the absorption area outside thereof.

According to an additional aspect of the invention, the inner layer may be made of a material composed of:
- natural fibres, optionally selected from the group comprising or consisting of celluloses, cellulose derivatives, cotton, bamboo, and combinations thereof, and/or
- synthetic polymeric fibres, optionally selected from the group comprising or consisting of polyethylene, polypropylene, two-component fibres, and combinations thereof, and/or
- combinations of said natural fibres and said synthetic polymeric fibres.

According to another aspect of the invention, the outer layer may be made of a laminate composite material formed from a non-woven fabric with a film of synthetic polymer fibres, optionally selected from the group comprising or consisting of polyethylene and/or polypropylene and/or combinations thereof, wherein the material of the outer layer is optionally breathable, whereby it is configured to let air, not liquid or body fluids, pass through.

It is still specific subject-matter of the present invention a method of manufacturing a urine absorbent bag as previously described comprising the following steps:
A. manufacturing the inner layer through a technique selected from the group comprising or consisting of carding, spunbond making techniques, spunlace making techniques, laminate and/or coupled and/or perforated sheet making techniques;
B. manufacturing the outer layer through a laminate material;
C. assembling the inner layer, the absorbent core and the outer layer to each other to form the planar multilayer structure through use of chemical binders and/or mechanical and/or thermal techniques;
D. folding the planar multilayer structure according to a "C"-shape, permanently closing the bottom edge through welding and/or adhesives to form the closed bottom, and permanently coupling the end portion of the second lateral edge of the planar multilayer structure onto the first lateral portion through welding and/or adhesives.

According to another aspect of the invention, the manufacturing method may further comprise the following step before step C:
E1. making in line (On Line) the absorbent core by dosing said at least one absorbent powder in the absorption area with varying weights, optionally in a continuous and/or stepped way, along the longitudinal axis and/or along an axis orthogonal to the longitudinal axis, wherein said at least one absorbent powder is then trapped between two layers of cellulosic material and united and held between them through hot melt adhesives; or
E2. making off line the absorbent core by introducing absorbent material containing said at least one absorbent powder.

According to an additional aspect of the invention, the manufacturing method may further comprise the following step before step C:
F. manufacturing the intermediate distribution layer through a technique selected from the group comprising or consisting of carding, spunbond making techniques, spunlace making techniques, laminate and/or coupled and/or perforated sheet making techniques;
wherein step C also assembles the intermediate distribution layer with the inner layer, the absorbent core and the outer layer.

According to another aspect of the invention, the manufacturing method may further comprise the following step after step C:
G. executing a patterning and/or embossing on the inner layer in correspondence of the absorption area to obtain said one or more first transfer pattern and/or embossing lines and/or said one or more second barrier pattern and/or embossing lines.

The urine absorbent bag according to the invention offers many advantages with respect to the prior art solutions.

First of all, it is wearable in a simple, adaptable and reliable way to protect and contain the penis.

Also, the urine absorbent bag according to the invention incorporates a plurality of materials with high absorbent properties, expandable on the basis of different capabilities to incorporate and retain urinary liquids, having a specific arrangement including a specific retention area of adequate capacity, thus allowing to absorb urine leakage in a reliable and efficient way.

Finally, the urine absorbent bag according to the invention is simple and inexpensive to manufacture.

The present invention will be now described, by way of illustration and not by way of limitation, according to its preferred embodiments, by particularly referring to the Figures of the annexed drawings, in which:
Figure 1 shows a plan view of a first embodiment of the urine absorbent bag according to the invention, in a first configuration;
Figure 2 shows a schematic front view of the absorbent bag of Figure 1;
Figure 3 shows a plan view of the planar multilayer structure of the absorbent bag of Figure 1, in which internal components are visible;
Figure 4 shows a section view along the transverse plane A-A' of Figure 3 (Fig. 4a) and a section view along the longitudinal plane B-B' of Figure 3 (Fig. 4b) of the absorbent bag of Figure 1;
Figure 5 shows a section view along the longitudinal plane B-B' of Figure 3 (Fig. 5a) of the absorbent core of the absorbent bag of Figure 1 and the section views along a similar longitudinal plane of the absorbent core of two different embodiments of the urine absorbent bag according to the invention (Fig. 5b and 5c);
Figure 6 shows a section view along the transverse plane A-A' of Figure 3 (Fig. 6a) of the absorbent core of the absorbent bag of Figure 1 and the section views along a similar transverse plane of the absorbent core of three different embodiments of the urine absorbent bag according to the invention (Fig. 6b, 6c and 6d);
Figure 7 shows a plan view of the planar multilayer structure of a second embodiment of the urine absorbent bag according to the invention, in which in which internal components are visible;
Figure 8 shows a plan view of the absorbent bag of Figure 1, in a second configuration;
Figure 9 shows a plan view of the planar multilayer structure of a third embodiment of the urine absorbent bag according to the invention, in which in which internal components are visible;
Figure 10 shows a plan view of the planar multilayer structure of a fourth embodiment (Fig. 10a), and an alternative embodiment thereof (Fig. 10b), of the urine absorbent bag according to the invention , in the second configuration;
Figure 11 shows a plan view of the planar multilayer structure of a fifth embodiment of the urine absorbent bag according to the invention, in the second configuration; and
Figure 12 shows a plan view of the planar multilayer structure of a sixth embodiment (Fig. 12a), and an alternative embodiment thereof (Fig. 12b), of the urine absorbent bag according to the invention, in the second configuration.

In the Figures identical reference numerals will be used for alike elements.

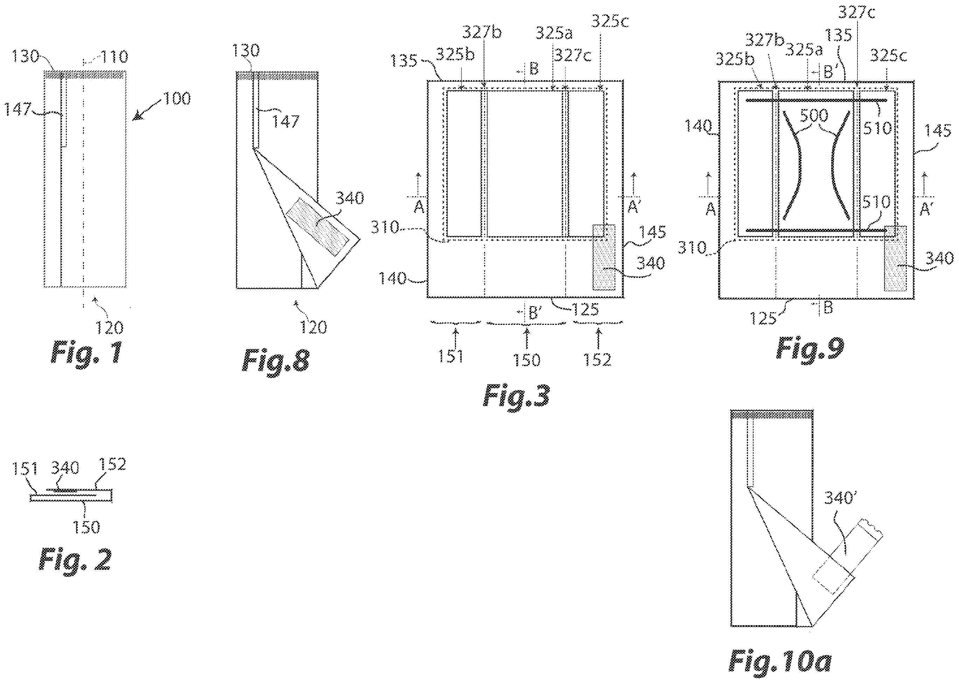

Referring to Figures 1-4, it can be observed that a preferred embodiment of the urine absorbent bag according to the invention has a planar multilayer structure, generally indicated with the reference numeral 100, folded in "C"-shape to form the bag having a longitudinal axis 110 (as shown in Figure 1), wherein the bag has a top opening 120 (i.e. positioned at the top end of the longitudinal axis 110 of the planar multilayer structure 100) defined by a top edge 125 of the planar multilayer structure 100, and a closed bottom 130 (positioned at the bottom end of the longitudinal axis 110 of the planar multilayer structure 100) defined by a bottom edge 135 of the planar multilayer structure 100. As shown in Figure 2, when the planar multilayer structure 100 is folded in "C"-shape to form the bag, a first lateral portion 151 thereof is overlapping a central portion 150 so that a first lateral edge 140 (that constitutes the end of the first lateral portion 151) is placed below a second lateral portion152 of the planar multilayer structure 100 the end of which constitutes a second lateral edge 145 thereof.

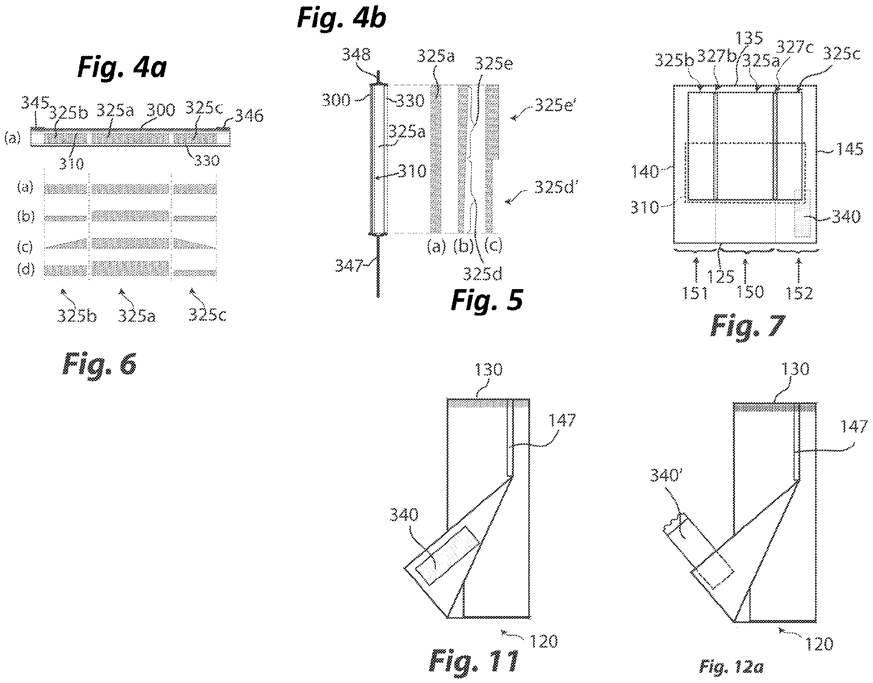

As shown in greater detail in Figure 4, the planar multilayer structure 100 of the preferred embodiment of the urine absorbent bag according to the invention comprises an inner layer 300 (usually indicated in the technical field of sanitary towels as "topsheet"), an intermediate distribution layer 310, an absorbent core, and an outer layer 330 (usually indicated in the technical field of sanitary towels as "backsheet"). The end portions of the outer layer 330 are coupled to the end portions of the inner layer 300, e.g. through hot melt adhesive and/or mechanical and/or thermal techniques, such as welding, e.g. ultrasonic welding, to form sealed end portions of the planar multilayer structure 100 so as to close and contain the intermediate distribution layer 310 and the absorbent core; in particular, the two sealed side end portions 345 and 346 are visible in Figure 4a, and the sealed top end portion 347 and the sealed bottom end portion 348 are visible in Figure 4b.

Lo inner layer 300 is made of a material composed of natural fibres and/or synthetic polymeric fibres and/or combinations of natural fibres and synthetic polymeric fibres. In particular, natural fibres can be selected from the group comprising or consisting of celluloses, cellulose derivatives, cotton, bamboo, and combinations thereof; synthetic polymeric fibres can be selected from the group comprising or consisting of polyethylene, polypropylene, bicomponent fibres (i.e. obtained from a mixture of at least two synthetic polymeric fibres), and combinations thereof. The inner layer 300 is configured to come into contact with a user's penis, when the penis is inserted into the bag 100, to quickly absorb the fluid (e.g. urine) removing it from the user's body during urination or leakage, remaining substantially dry and also ensuring the softness and fluffiness del bag 100 of the bag 100 when it comes into contact with the penis of the user in both wet and dry conditions. The technologies employed for the manufacture of the inner layer 300 may vary according to the specific desired arrangement of the planar multilayer structure, by using production processes used in the health and hygiene field such as carding, spunbond making techniques (i.e. polypropylene non-woven fabric), spunlace making techniques (i.e. synthetic polymeric fibre non-woven fabric), laminate and/or coupled and/or perforated sheet making techniques.

The outer layer 330 is made of a laminate composite material formed from a non-woven fabric with a film of synthetic polymer fibres, such as polyethylene and/or polypropylene and/or combinations thereof. The material of the outer layer 330 can be breathable, and in this case the outer layer 330 is configured to let only air, not leakage of liquids or body fluids of a user (e.g. urine), pass through, thus allowing to reduce the internal temperature of the product. In other embodiments of the urine absorbent bag according to the invention, the material of the outer layer 330 can be non-breathable.

The absorbent core is interposed between the inner layer 300 and the outer layer 330 and it is configured to absorb and retain fluid. As shown in Figure 3, the absorbent core comprises a liquid and fluid absorption area 325, made of a superabsorbent polymer powder or SAP, possibly mixed with odour-absorbing powders (to reduce and hold the bad odour given off by the body fluid), that is trapped between two films of cellulosic material and that is contained within these two films of cellulosic material, whereby the edges of the absorption area 325 are positioned inside the planar multilayer structure 100 (and spaced from the top edge 125, the bottom edge 135 and the two lateral edges 140 and 145 of the planar multilayer structure 100). In particular, the absorption area 325 is composed of three absorption sub-areas 325a, 325b and 325c extending parallel to the longitudinal axis 110 del urine absorbent bag, in which two longitudinal flow channels 327b and 327c separate (in a direction transverse to the longitudinal axis 110) the central sub-area 325a from the two lateral sub-areas 325b and 325c, respectively. Consequently, such longitudinal flow channels 327b and 327c are devoid of SAP, permitting an easy folding, in correspondence with them, of the "C"-shaped planar multilayer structure 100 (as shown in Figure 2), improving wearability and comfort of the urine absorbent bag according to the invention; in particular, the two lateral sub-areas 325b and 325c are arranged in the first lateral portion 151 and in the second lateral portion 152, respectively, of the planar multilayer structure 100, while the central sub-area 325a is arranged in the central portion 150 of the multilayer structure planar 100. Optionally, such longitudinal flow channels 327b and 327c, devoid of SAP, are continuous and extend longitudinally for the extension of the entire absorption area 325.

In particular, other embodiments of the urine absorbent bag according to the invention can be devoid of longitudinal flow channels or can have more than two longitudinal flow channels in the absorption area 325, whereby the latter can be composed by more than three absorption sub-areas extending parallel to the longitudinal axis 110 of the urine absorbent bag.

Figures 4a and 4b show section views of the absorbent bag of Figure 1 along the transverse plane A-A' of Figure 3 (Fig. 4a), that is along plane A-A' orthogonal to the longitudinal axis 110 of the planar multilayer structure 100, and a section view along the longitudinal plane B-B' of Figure 3 (Fig. 4b), that is along plane B-B' parallel to the longitudinal axis 110 of the planar multilayer structure 100. As shown in Figures 4a and 4b, the SAP content in the absorption area 325 of the absorbent bag of Figure 1, and consequently the thickness of the absorption area 325, is constant.

Other embodiments of the urine absorbent bag according to the invention may have the SAP content in the absorption area 325 (and consequently the thickness of the absorption area 325), that is variable, optionally continuously, along the direction parallel and/or along the direction transverse to the longitudinal axis 110 of the urine absorbent bag according to the invention.

By way of illustration and not by way of limitation, Figure 5 shows:
- a section view along the longitudinal plane B-B' of Figure 3 of the absorbent core of the absorbent bag of Figure 1, in which the thickness is constant (Fig. 5a);
- a section view along a longitudinal plane (similar to the longitudinal plane B-B' of Figure 3) of the absorbent core of a different embodiment of the absorbent bag according to the invention, in which the thickness is variable in a continuous way from a zone 325d with reduced absorption to a storage zone 325e, wherein the zone 325d with reduced absorption is closer to the top edge 125 (and to the top opening 120 of the urine absorbent bag according to the invention) with respect to the storage area 325e (Fig. 5b), whereby the zone 325d with reduced absorption is more distant from the bottom edge 135 (and from the closed bottom 130 of the urine absorbent bag according to the invention) with respect to the storage zone 325e; in this case, the absorption area 325 is divided into two zones of substantially equal extension in the plane of the planar multilayer structure 100: the zone 325d with reduced absorption and the storage zone 325e;
- a section view along a longitudinal plane (similar to the longitudinal plane B-B' of Figure 3) of the absorbent core of a further different embodiment of the absorbent bag according to the invention, in which the thickness is variable in a stepped way from a zone 325d' with reduced absorption, to a storage zone 325e', wherein the zone 325d' with reduced absorption is closer to the top edge 125 (and to the top opening 120 of the urine absorbent bag according to the invention) with respect to the storage zone 325e', and wherein the thickness of the zone 325d' with reduced absorption is constant and is lower than the constant thickness of the storage zone 325e' (Fig. 5c).

Other embodiments may have both continuous and stepped variability of the SAP content in the absorption area 325 (and consequently of the thickness of the absorption area 325) along the direction parallel to the longitudinal axis 110 of the urine absorbent bag according to the invention.

Still by way of illustration and not by way of limitation, Figure 6 shows:
- a section view along the transverse plane A-A' of Figure 3 of the absorbent core of the absorbent bag of Figure 1, in which the thickness is constant in the three absorption sub-areas 325a, 325b and 325c (Fig. 6a);
- a section view along a transverse plane (similar to transverse plane A-A' of Figure 3) of the absorbent core of a different embodiment of the absorbent bag according to the invention, in which the thickness is variable in a stepped way from the two lateral sub-areas 325b and 325c to the central sub-area 325a, wherein the thickness is the same in the two lateral sub-areas 325b and 325c, where it is constant and lower than the constant thickness of the central sub-area 325a (Fig. 6b);
- a section view along a transverse plane (similar to transverse plane A-A' of Figure 3) of the absorbent core of a further different embodiment del absorbent bag according to the invention, wherein the thickness in the two lateral sub-areas 325b and 325c is variable in a continuous and it increases from the distal end (with respect to the central sub-area 325a) to the proximal end (with respect to the central sub-area 325a), and wherein the thickness of the central sub-area 325a is constant and not lower than the maximum thickness in the two lateral sub-areas 325b and 325c, whereby the arrangement of the two lateral sub-areas 325b and 325c is symmetrical (Fig. 6c); and
- a section view along a transverse plane (similar to transverse plane A-A' of Figure 3) of the absorbent core of a still further different embodiment of the absorbent bag according to the invention, wherein the thickness is variable in a stepped way from the two lateral sub-areas 325b and 325c to the central sub-area 325a, wherein the thickness of the first lateral sub-area 325b is constant and lower than the thickness of the second lateral sub-area 325c, that in turn is constant and lower than the constant thickness of the central sub-area 325a (Fig. 6d - in other embodiments, the thickness of the first lateral sub-area 325b may be greater than the thickness of the second lateral sub-area 325c).

It should be noted that further embodiments of the absorbent bag according to the invention may have any combination among the arrangements shown in Figure 5 with those shown in Figure 6, and also arrangements other than those shown, variable in a continuous and/or stepped way.

In the embodiments where the SAP content in the absorption area 325 (and consequently the thickness of the absorption area 325) is variable as shown in Figures 5b and 5c, the SAP content in the storage zone 325e (or 325e') is variable from 60% to 90%, optionally equal to at least 70%, of the amount of SAP of the entire absorption area 325. This different distribution of SAP, the amount of which is higher in the storage zone 325e (or 325e') with respect to the zone 325d (or 325d') with reduced absorption, allows to have a guided control of the body fluid (e.g. urine), by significantly reducing the liquid content in the zone 325d (or 325d') with reduced absorption that remains substantially dry, helping to keep the portion of the inner layer 300 that is in contact with the user's penis dry.

Also, the two (or more) longitudinal flow channels 327b and 327c, devoid of SAP (and other absorbent powders) promote (substantially by gravity and/or pressure and/or capillarity) the rapid transfer of body fluid (e.g. urine) from the zone 325d (or 325d') with reduced absorption to the storage zone 325e (or 325e').

In particular, thanks to the presence of SAP, the absorbent core has a weight ranging from 50% to 75%, optionally not more than 70%, of the weight of the entire planar multilayer structure 100.

In a first embodiment of the method of manufacturing the urine absorbent bag according to the invention, the absorbent core is manufactured in line (On Line) through a system of dosing SAP that is dosed in the absorption area 325. The SAP is then trapped between two layers of cellulosic material and joined and held between them through hot melt adhesives. The application and dosage of SAP take place on the entire surface of the absorption area 325 with interruption of the two (or more) longitudinal flow channels 327b and 327c. Furthermore, in combination with or alternatively to SAP, other absorbent powders can be applied, and in combination with SAP and/or other absorbent powders odour-absorbing powders can be applied; in particular, the odour-absorbing powders can be incorporated into SAP and/or other absorbent powders or mixed with them. In the case of the embodiments in which the SAP content in the absorption area 325 is varying along the parallel direction and/or along the transverse direction, as for example shown in Figures 5b and 5c and/or 6b, 6c and 6d, SAP is dosed in the absorption area 325 with varying weights (in grams) in a variable way, optionally in a continuous and/or stepped way, along the direction parallel to the longitudinal axis 110 of the absorbent bag for urine, so as to form the zone 325d (or 325d') with reduced absorption and the storage zone 325e (or 325e').

In a second embodiment of the method of manufacturing the urine absorbent bag according to the invention, the absorbent core is made Off Line with the introduction of absorbent material containing SAP and/or other absorbent powders and possibly odour-absorbing powders. Also in this case, the odour-absorbing powders can be incorporated into SAP and/or other absorbent powders or mixed with them.

Still referring to Figure 3, the intermediate distribution layer 310 is interposed between the inner layer 300 and the absorbent core, it has an extension in the plane of the planar multilayer structure 100 corresponding to the absorbent core and it is in a position facing thereto; optionally, the intermediate distribution layer 310 extends in the plane of the planar multilayer structure 100 so as to protrude beyond the edges of the absorption area 325. Such intermediate distribution layer 310 is configured to acquire and distribute body fluids (e.g. urine) towards the absorption area 325 (optionally towards the zone 325d or 325d' with reduced absorption, if any, and, in this case, more optionally also partially towards the storage area 325e or 325e'), allowing the inner layer 300 to remain dry in contact with the penis of the user, consequently avoiding possible flushing and/or burning problems, and it is optionally further configured to temporarily retain body fluids and give sufficient time to the two longitudinal flow channels 327b and 327c (and to the zone 325d or 325d' with reduced absorption, if any) di to transfer fluids to the absorption area 325 (namely in the storage zone 325e or 325e', if any) per for total absorption (whereby even the zone 325d or 325d' with reduced absorption, if any, is maintained substantially dry). In particular, when it absorbs body fluid (namely in the storage zone 325e or 325e', if any), the absorbent core has a corresponding increase in volume creating a sort of self-holding around the penis of the user.

Referring to Figure 7, it can be observed that a second embodiment of the urine absorbent bag according to the invention has a multilayer structure in which the intermediate distribution layer 310' is interposed between the inner layer 300 and the absorbent core in correspondence of only a part of the absorbent core, that is closer to the top edge 125 (and to the top opening 120 of the urine absorbent bag according to the invention); if present, the zone 325d (or 325d') with reduced absorption is the part of the absorbent core in correspondence of which the intermediate distribution layer 310' is arranged. Optionally, the intermediate distribution layer 310' extends in the plane of the planar multilayer structure 100 so as to protrude beyond the edges of the absorption area 325, with the exception of the edge of the intermediate layer 310' facing the bottom edge 135 of the planar multilayer structure 100.

Such intermediate distribution layer 310 (or 310') can be made of a material composed of natural fibres and/or synthetic polymeric fibres and/or combinations of natural fibres and synthetic polymeric fibres. In particular, natural fibres are optionally selected from the group comprising or consisting of celluloses, cellulose derivatives, viscose, bamboo, and combinations thereof; synthetic polymeric fibres are optionally selected from the group comprising or consisting of polyethylene, polypropylene, polyester, bicomponent fibres (i.e. obtained from a mixture of at least two synthetic polymeric fibres), and combinations thereof.

The method of manufacturing the intermediate distribution layer 310 (or 310') is similar to the one for manufacturing the inner layer 300.

The inner layer 300 is provided with a longitudinal adhesive label 340, that is optionally repositionable, operating as an (optionally multiple) opening and closure system for the urine absorbent bag according to the invention. Referring to Figures 2-4, when the planar multilayer structure 100 is folded in "C"-shape to form the bag, the longitudinal adhesive label 340 is configured to couple (optionally in a removable way) the second lateral section 152 onto the first lateral section 151, as shown in Figure 8. In particular, the longitudinal adhesive label 340 is positioned in proximity of the second lateral edge 145 (i.e. in correspondence of the second lateral portion 152) and is closer to the top edge 125 with respect to the bottom edge 135 of the planar multilayer structure 100. Thanks to the easy and adjustable positioning of the product, the longitudinal adhesive label 340 allows a simplicity of use.

In the preferred embodiment of the method of manufacturing the urine absorbent bag according to the invention, the inner layer 300, the intermediate distribution layer 310, the absorbent core and the outer layer 330 are assembled to each other to form the planar multilayer structure 100 through use of chemical binders such as hot melt adhesives and/or mechanical and/or thermal techniques, such as welding, e.g. ultrasonic welding.

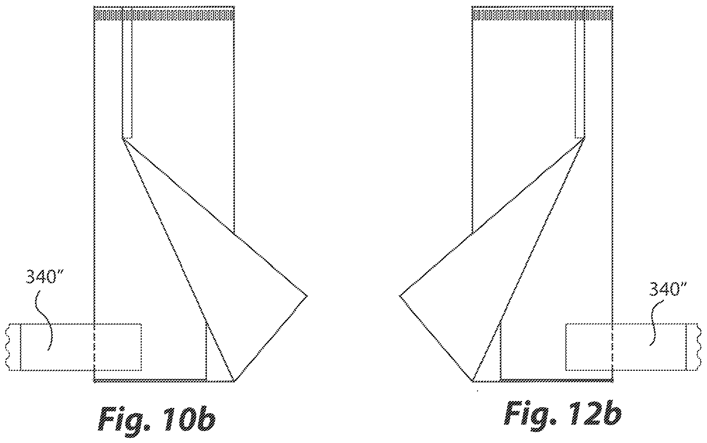

As shown in Figure 9, a third embodiment of the method of manufacturing comprises a step of patterning and/or embossing on the inner layer 300 in correspondence of the absorption area 325. Such patterning and/or embossing step produces two first transfer lines 500, optionally in correspondence of the central sub-area 325a, which extend mainly in a direction parallel to the longitudinal axis 110 of the planar multilayer structure 100, which can be curvilinear (and not necessarily rectilinear), as in the fourth embodiment of the urine absorbent bag according to the invention shown in Figure 10, for a longitudinal length optionally not smaller than 60% of the longitudinal length of the absorption area 325. These two first transfer lines 500 (which are actually barriers operating as transfer lines since the flow is substantially parallel to them) are configured to guide body fluid (e.g. urine) towards the closed bottom 130 of the urine absorbent bag (namely from the zone 325d or 325d' with reduced absorption to the liquid storage zone 325e or 325e', if any). Furthermore, such additional step produces two second barrier lines 510 extending along a direction transverse to the longitudinal axis 110 of the planar multilayer structure 100 in correspondence of the transverse edges of the absorption area 325, optionally covering the entire central sub-area, the two longitudinal flow channels 327b and 327c and at least partially the two lateral sub-areas 325b and 325c, more optionally for a transverse length (i.e. for a width) not lower than 70% of the transverse length (i.e. of the width) of the absorption area 325; such two transverse barrier lines 510 are configured to operate as protection barrier to prevent the leakage of body fluid stored in the absorption area 325 outside thereof and, consequently, outside the urine absorbent bag according to the invention.

The patterning and/or embossing step allows to join the inner layer 300 with the intermediate distribution layer 310 with greater reliability.

Other embodiments of the manufacturing method according to the invention may have a different number of transfer lines and/or barrier lines, and/or they may be devoid of transfer lines and/or barrier lines.

Further embodiments of the manufacturing method according to the invention may not have the patterning and/or embossing step.

Finally, the manufacturing method according to the invention comprises a final step of arranging the planar multilayer structure 100 to form the absorbent bag. As shown in Figures 1 and 2, the planar multilayer structure 100 is folded in "C"-shape, by using the two longitudinal flow channels 327b and 327c as folding lines, and the bottom edge 135 is permanently closed through welding (optionally ultrasound welding) and/or adhesives (optionally hot melt adhesives) to form the closed bottom 130. Also, an end portion 147 of the second lateral edge 145 of the planar multilayer structure 100 (constituted by the end of the second lateral portion 152), longitudinally extending from the bottom edge 135 for a length not longer than 50%, optionally not longer than 40%, of the length of the second lateral edge 145, is permanently coupled onto the first lateral portion 152 through welding (optionally ultrasound welding) and/or adhesives (optionally hot melt adhesives).

As shown in Figure 8, the longitudinal adhesive label 340 is configured to couple, optionally in a removable way, the second lateral portion 152 onto the first lateral portion 151, making an adjustable closure system, optionally a repositionable one (in this case the second longitudinal adhesive label 340 can be a so-called "binder band"), configured to better manage the use of the absorbent bag in use and/or for subsequent checks of the condition of the absorbent bag. In other words, the specific "C"-shaped allows a user to wear the urine absorbent bag according to the invention, adjusting the adherence as desired, respecting the anatomy and comfort needs of each user, through overlapping of the lateral portions 151 and 152 kept in the closed position by the longitudinal adhesive label 340. In particular, in the case where such longitudinal adhesive label 340 is repositionable, it easily allows to wear, position, adjust and remove the absorbent urine bag according to the invention.

As shown in Figure 10a, in a third embodiment of the urine absorbent bag according to the invention, the outer layer 330 is provided with an elastic adhesive label 340', that is elastically extendable, positioned in proximity of the second lateral edge 145 (i.e. in correspondence of the second lateral portion 152) and closer to the top edge 125 than the bottom edge 135 of the planar multilayer structure 100; this elastic adhesive label 340' is configured to couple, optionally in a removable way, the second lateral portion 152 onto the first lateral portion 151. In an alternative embodiment of the urine absorbent bag according to the invention, shown in Figure 10b, the outer layer 330 is provided with an elastic adhesive label 340", that is elastically extensible, positioned in proximity of the first lateral edge 140 (i.e. in correspondence of the first lateral portion 151) and closer to the top edge 125 than the bottom edge 135 of the planar multilayer structure 100; this elastic adhesive label 340' is also configured to couple, optionally in a removable way, the second lateral portion 152 onto the first lateral portion 151.

It should be noted that other embodiments of the urine absorbent bag according to the invention may have, instead of the adhesive label 340 or 340', removable coupling devices with hooks and loops (of the type known with the commercial name Velcro^{®}) arranged onto the inner layer 300 and the outer layer 330. Moreover, further embodiments of the urine absorbent bag according to the invention may have, onto the inner layer 300, instead of the adhesive label 340 or 340', an area provided with hooks that directly couples onto the outer layer 330.

It should be noted that other embodiments of the urine absorbent bag according to the invention may have the planar multilayer structure only having the inner layer 300, the outer layer 330 and the absorbent core interposed between the inner layer 300 and the outer layer 330, wherein the absorbent core has an extension in the plane of the planar multilayer structure slightly lower than that of the inner layer 300 and outer layer 330, and wherein the planar multilayer structure is devoid of the intermediate distribution layer 310. Similarly to what shown in Figures 1 and 8-10, in these other embodiments of the urine absorbent bag according to the invention, the bottom edge 135 is permanently closed to form the closed bottom 130 and the end portion 147 of the second lateral edge 145 of the planar multilayer structure (constituted by the end of the second lateral portion 152) is permanently coupled to the first lateral portion 152.

In particular, the embodiments shown in Figures 1-10 have a closure towards the left.

It should be noted that other embodiments of the urine absorbent bag according to the invention can have a closure towards the right. By way of illustration, and not by way of limitation, Figure 11 shows a fifth embodiment (similar to that of Figure 1) in which the urine absorbent bag according to the invention is provided with a longitudinal adhesive label 340 configured to implement a closure towards the right, Figure 12a shows a sixth embodiment (similar to that of Figure 10a) in which the urine absorbent bag according to the invention is provided with an elastic adhesive label 340' configured to implement a closure towards the right, and Figure 12b shows a further embodiment (similar to that of Figure 10b) in which the urine absorbent bag according to the invention is provided with an elastic adhesive label 340" configured to implement a closure towards the right.

The preferred embodiments of this invention have been described and a number of variations have been suggested hereinbefore, but it should be understood that those skilled in the art can make other variations and changes without so departing from the scope of protection thereof, as defined by the attached claims.

## Claims

1. Urine absorbent bag configured to house a penis of a user, having a planar multilayer structure (100) folded in "C"-shape to form said absorbent bag having a longitudinal axis (110), wherein said absorbent bag has a top opening (120), configured to receive the penis of the user, that is defined by a top edge (125) of the planar multilayer structure (100), wherein said absorbent bag further has a closed bottom (130) defined by a bottom edge (135) of the planar multilayer structure (100), whereby a first lateral portion (151) of the planar multilayer structure (100) is overlapping a central portion (150) of the planar multilayer structure (100) so that a first lateral edge (140) thereof is placed below a second lateral portion (152) of the planar multilayer structure (100), wherein the planar multilayer structure (100) comprises an inner layer (300) configured to come into contact with the penis of the user, an outer layer (330) and an absorbent core interposed between the inner layer (300) and the outer layer (330), wherein the absorbent core is configured to absorb and retain fluid, wherein an end portion (147) of a second lateral edge (145) of the planar multilayer structure (100), longitudinally extending from the bottom edge (135) to a length not greater than 50% of the length of the second lateral edge (145), is permanently coupled onto the first lateral portion (151).

2. Absorbent bag according to claim 1, wherein the end portion (147) of the second lateral edge (145) of the planar multilayer structure (100) longitudinally extends from the bottom edge (135) to a length not greater than 40% of the length of the second lateral edge (145).

3. Absorbent bag according to claim 1 or 2, wherein the inner layer (300) or the outer layer (330) is provided with an adhesive label (340; 340'; 340") configured to adjustably couple the second lateral edge (152) onto the first lateral portion (151), wherein the adhesive label (340; 340'; 340") is optionally repositionable and/or extensible.

4. Absorbent bag according to any one of the preceding claims, wherein the absorbent core comprises a liquid and fluid absorption area (325) made of at least one absorbent powder, optionally a superabsorbent polymer powder or SAP, trapped between two films of cellulosic material, wherein an amount of said at least one absorbent powder in the absorption area (325) is varying, optionally in a continuous and/or stepped way:
- along the longitudinal axis (110), whereby the absorption area (325) is divided into two zones, optionally of equal extension in the plane of the planar multilayer structure (100), consisting in a zone (325d; 325d') with reduced absorption and in a storage zone (325e; 325e'), wherein the zone (325d; 325d') with reduced absorption is closer to the top edge (125) with respect to the storage zone (325e; 325e'), wherein the amount of said at least one absorbent powder is higher in the storage zone (325e; 325e') with respect to the zone (325d; 325d') with reduced absorption; and/or
- along an axis orthogonal to the longitudinal axis (110).

5. Absorbent bag according to claim 4, wherein the amount of said at least one absorbent powder in the absorption area (325) is varying along the longitudinal axis (110) and the amount of said at least one absorbent powder in the storage zone (325e; 325e') is variable from 60% to 90%, optionally equal to at least 70%, of the amount of said at least one absorbent powder in the entire absorption area (325).

6. Absorbent bag according to any one of the preceding claims, wherein the absorption area (325) is composed of at least three absorption sub-areas (325a, 325b, 325c) extending parallel to the longitudinal axis (110), wherein at least two longitudinal flow channels (327b, 327c) devoid of said at least one absorbent powder separate said at least three absorption sub-areas (325a, 325b, 325c) from each other.

7. Absorbent bag according to any one of the preceding claims, wherein the planar multilayer structure (100) further comprises an intermediate distribution layer (310) interposed between the inner layer (300) and the absorbent core, wherein the intermediate distribution layer (310) is configured to acquire and distribute body fluids towards the absorption area (325).

8. Absorbent bag according to claim 7, wherein the intermediate distribution layer (310) is made of a material composed of:
- natural fibres, optionally selected from the group comprising or consisting of celluloses, cellulose derivatives, viscose, bamboo, and combinations thereof, and/or
- synthetic polymeric fibres, optionally selected from the group comprising or consisting of polyethylene, polypropylene, polyester, two-component fibres, and combinations thereof, and/or
- combinations of said natural fibres and said synthetic polymeric fibres.

9. Absorbent bag according to any one of the preceding claims, wherein the inner layer (300) is provided with:
- one or more, optionally two, first transfer pattern and/or embossing lines (500) extending along a direction parallel to the longitudinal axis (110) to a longitudinal length optionally not smaller than 60% of the longitudinal length of the absorption area (325), wherein said one or more first transfer pattern and/or embossing lines (500) are configured to guide a body fluid towards the closed bottom (130), and/or
- one or more, optionally two, second barrier pattern and/or embossing lines (510) extending along a direction transverse to the longitudinal axis (110), optionally in correspondence with transverse edges of the absorption area (325), optionally for a transverse length not smaller than 70% of the transverse length of the absorption area (325), wherein said one or more second barrier pattern and/or embossing lines (510) are configured to operate as protection barrier to prevent leakage of body fluids stored in the absorption area (325) outside thereof.

10. Absorbent bag according to any one of the preceding claims, wherein the inner layer (300) is made of a material composed of:
- natural fibres, optionally selected from the group comprising or consisting of celluloses, cellulose derivatives, cotton, bamboo, and combinations thereof, and/or
- synthetic polymeric fibres, optionally selected from the group comprising or consisting of polyethylene, polypropylene, two-component fibres, and combinations thereof, and/or
- combinations of said natural fibres and said synthetic polymeric fibres.

11. Absorbent bag according to any one of the preceding claims, wherein the outer layer (330) is made of a laminate composite material formed from a non-woven fabric with a film of synthetic polymer fibres, optionally selected from the group comprising or consisting of polyethylene and/or polypropylene and/or combinations thereof, wherein the material of the outer layer (330) is optionally breathable, whereby it is configured to let air, not liquid or body fluids, pass through.

12. Method of manufacturing a urine absorbent bag according to any one of claims 1 to 11 comprising the following steps:
A. manufacturing the inner layer (300) through a technique selected from the group comprising or consisting of carding, spunbond making techniques, spunlace making techniques, laminate and/or coupled and/or perforated sheet making techniques;
B. manufacturing the outer layer (330) through a laminate material;
C. assembling the inner layer (300), the absorbent core and the outer layer (330) to each other to form the planar multilayer structure (100) through use of chemical binders and/or mechanical and/or thermal techniques;
D. folding the planar multilayer structure (100) according to a "C"-shape, permanently closing the bottom edge (135) through welding and/or adhesives to form the closed bottom (130), and permanently coupling the end portion (147) of the second lateral edge (145) of the planar multilayer structure (100) onto the first lateral portion (152) through welding and/or adhesives.

13. Method of manufacturing according to claim 12, wherein the urine absorbent bag is the absorbent bag according to claim 4 or 5, or according to any one of claims 6-11, when depending on claim 4, further comprising the following step before step C:
E1. making in line (On Line) the absorbent core by dosing said at least one absorbent powder in the absorption area (325) with varying weights, optionally in a continuous and/or stepped way, along the longitudinal axis (110) and/or along an axis orthogonal to the longitudinal axis (110), wherein said at least one absorbent powder is then trapped between two layers of cellulosic material and united and held between them through hot melt adhesives; or
E2. making off line the absorbent core by introducing absorbent material containing said at least one absorbent powder.

14. Method of manufacturing according to claim 13, wherein the urine absorbent bag is the absorbent bag according to any one of claims 7 or 8, or according to any one of claims 9 to 11, when depending on claim 7, further comprising the following step before step C:
F. manufacturing the intermediate distribution layer (310) through a technique selected from the group comprising or consisting of carding, spunbond making techniques, spunlace making techniques, laminate and/or coupled and/or perforated sheet making techniques;
wherein step C also assembles the intermediate distribution layer (310) with the inner layer (300), the absorbent core and the outer layer (330).

15. Method of manufacturing according to claim 14, wherein the urine absorbent bag is the absorbent bag according to claim 9 or according to claim 10 or 11, when depending on claim 9, further comprising the following step after step C:
G. executing a patterning and/or embossing on the inner layer (300) in correspondence of the absorption area (325) to obtain said one or more first transfer pattern and/or embossing lines (500) and/or said one or more second barrier pattern and/or embossing lines.

## Patentansprüche

1. Urinabsorbierender Beutel, der so konfiguriert ist, dass er einen Penis eines Benutzers aufnehmen kann, mit einer ebenen mehrlagigen Struktur (100), die in "C"-Form gefaltet ist, um den absorbierenden Beutel mit einer Längsachse (110) zu bilden, wobei der absorbierende Beutel eine obere Öffnung (120) aufweist, die so konfiguriert ist, dass sie den Penis des Benutzers aufnimmt, und die durch einen oberen Rand (125) der ebenen mehrlagigen Struktur (100) definiert ist, wobei der absorbierende Beutel ferner einen geschlossenen Boden (130) aufweist, der durch einen unteren Rand (135) der ebenen mehrlagigen Struktur (100) definiert ist, wobei ein erster seitlicher Abschnitt (151) der ebenen mehrlagigen Struktur (100) einen zentralen Abschnitt (150) der ebenen mehrlagigen Struktur (100) überlappt, so dass ein erster seitlicher Rand (140) davon unter einem zweiten seitlichen Abschnitt (152) der ebenen mehrlagigen Struktur (100) angeordnet ist, wobei die planare mehrschichtige Struktur (100) eine innere Schicht (300), die so konfiguriert ist, dass sie mit dem Penis des Benutzers in Kontakt kommt, eine äußere Schicht (330) und einen absorbierenden Kern, der zwischen der inneren Schicht (300) und der äußeren Schicht (330) angeordnet ist, umfasst, wobei der absorbierende Kern so konfiguriert ist, dass er Flüssigkeit absorbiert und zurückhält, wobei ein Endabschnitt (147) eines zweiten seitlichen Randes (145) der ebenen mehrlagigen Struktur (100), der sich in Längsrichtung von dem unteren Rand (135) bis zu einer Länge von nicht mehr als 50% der Länge des zweiten seitlichen Randes (145) erstreckt, dauerhaft mit dem ersten seitlichen Abschnitt (151) verbunden ist.

2. Absorbierender Beutel nach Anspruch 1, wobei sich der Endabschnitt (147) der zweiten Seitenkante (145) der ebenen Mehrschichtstruktur (100) in Längsrichtung von der Bodenkante (135) bis zu einer Länge von nicht mehr als 40% der Länge der zweiten Seitenkante (145) erstreckt.

3. Absorbierender Beutel nach Anspruch 1 oder 2, wobei die innere Schicht (300) oder die äußere Schicht (330) mit einem Klebeetikett (340; 340'; 340") versehen ist, das so konfiguriert ist, dass es den zweiten seitlichen Rand (152) einstellbar an den ersten seitlichen Abschnitt (151) koppelt, wobei das Klebeetikett (340; 340'; 340") optional repositionierbar und/oder dehnbar ist.

4. Absorbierender Beutel nach einem der vorhergehenden Ansprüche, wobei der Absorptionskern einen Flüssigkeits- und Fluidabsorptionsbereich (325) umfasst, der aus mindestens einem Absorptionspulver, optional einem superabsorbierenden Polymerpulver oder SAP, hergestellt ist, das zwischen zwei Filmen aus Zellulosematerial eingeschlossen ist, wobei eine Menge des mindestens einen Absorptionspulvers in dem Absorptionsbereich (325) variiert, optional in kontinuierlicher und/oder abgestufter Weise:
- entlang der Längsachse (110), wobei der Absorptionsbereich (325) in zwei Zonen unterteilt ist, die gegebenenfalls eine gleiche Ausdehnung in der Ebene der ebenen Mehrschichtstruktur (100) haben, bestehend aus einer Zone (325d; 325d') mit reduzierter Absorption und einer Speicherzone (325e; 325e'), wobei die Zone (325d; 325d') mit verringerter Absorption in Bezug auf die Speicherzone (325e; 325e') näher am oberen Rand (125) liegt, wobei die Menge des mindestens einen absorbierenden Pulvers in der Speicherzone (325e; 325e') in Bezug auf die Zone (325d; 325d') mit verringerter Absorption höher ist; und/oder
- entlang einer Achse orthogonal zur Längsachse (110).

5. Absorbierender Beutel nach Anspruch 4, wobei die Menge des mindestens einen Absorptionspulvers in dem Absorptionsbereich (325) entlang der Längsachse (110) variiert und die Menge des mindestens einen Absorptionspulvers in der Speicherzone (325e; 325e') von 60 % bis 90 %, optional gleich mindestens 70 %, der Menge des mindestens einen Absorptionspulvers in dem gesamten Absorptionsbereich (325) variabel ist.

6. Absorbierender Beutel nach einem der vorhergehenden Ansprüche, wobei der Absorptionsbereich (325) aus mindestens drei Absorptions-Teilbereichen (325a, 325b, 325c) besteht, die sich parallel zur Längsachse (110) erstrecken, wobei mindestens zwei Längsströmungskanäle (327b, 327c) ohne das mindestens eine absorbierende Pulver die mindestens drei Absorptions-Teilbereiche (325a, 325b, 325c) voneinander trennen.

7. Absorbierender Beutel nach einem der vorhergehenden Ansprüche, wobei die planare Mehrschichtstruktur (100) ferner eine Zwischenverteilungsschicht (310) umfasst, die zwischen der inneren Schicht (300) und dem absorbierenden Kern angeordnet ist, wobei die Zwischenverteilungsschicht (310) so konfiguriert ist, dass sie Körperflüssigkeiten aufnimmt und in Richtung des Absorptionsbereichs (325) verteilt.

8. Absorbierender Beutel nach Anspruch 7, wobei die Zwischenverteilungsschicht (310) aus einem Material hergestellt ist, das aus:
- Naturfasern, gegebenenfalls ausgewählt aus der Gruppe, die Cellulosen, Cellulosederivate, Viskose, Bambus und Kombinationen davon umfasst oder daraus besteht, und/oder
- synthetische Polymerfasern, gegebenenfalls ausgewählt aus der Gruppe, die Polyethylen, Polypropylen, Polyester, Zweikomponentenfasern und Kombinationen davon umfasst oder daraus besteht, und/oder
- Kombinationen aus den genannten Naturfasern und den genannten synthetischen Polymerfasern.

9. Absorbierender Beutel nach einem der vorangehenden Ansprüche, wobei die Innenschicht (300) mit versehen ist:
- eine oder mehrere, optional zwei, erste Transfermuster- und/oder Prägelinien (500), die sich entlang einer Richtung parallel zur Längsachse (110) bis zu einer Längslänge erstrecken, die optional nicht kleiner als 60 % der Längslänge des Absorptionsbereichs (325) ist, wobei das eine oder die mehreren ersten Transfermuster- und/oder Prägelinien (500) so konfiguriert sind, dass sie ein Körperfluid zum geschlossenen Boden (130) leiten, und/oder
- ein oder mehrere, optional zwei, zweite Barrieremuster und/oder Prägelinien (510), die sich entlang einer Richtung quer zur Längsachse (110) erstrecken, optional in Übereinstimmung mit den Querkanten des Absorptionsbereichs (325), optional über eine Querlänge, die nicht kleiner als 70% der Querlänge des Absorptionsbereichs (325) ist, wobei das eine oder die mehreren zweiten Barrieremuster und/oder Prägelinien (510) so konfiguriert sind, dass sie als Schutzbarriere wirken, um ein Auslaufen von Körperflüssigkeiten, die in dem Absorptionsbereich (325) gelagert sind, außerhalb davon zu verhindern.

10. Absorbierender Beutel nach einem der vorhergehenden Ansprüche, wobei die Innenschicht (300) aus einem Material besteht, das aus:
- Naturfasern, gegebenenfalls ausgewählt aus der Gruppe, die Cellulosen, Cellulosederivate, Baumwolle, Bambus und Kombinationen davon umfasst oder daraus besteht, und/oder
- synthetische Polymerfasern, gegebenenfalls ausgewählt aus der Gruppe, die Polyethylen, Polypropylen, Zweikomponentenfasern und Kombinationen davon umfasst oder daraus besteht, und/oder
- Kombinationen aus den genannten Naturfasern und den genannten synthetischen Polymerfasern.

11. Absorbierender Beutel nach einem der vorhergehenden Ansprüche, wobei die äußere Schicht (330) aus einem Laminat-Verbundmaterial hergestellt ist, das aus einem Vliesstoff mit einem Film aus synthetischen Polymerfasern gebildet ist, die optional aus der Gruppe ausgewählt sind, die Polyethylen und/oder Polypropylen und/oder Kombinationen davon umfasst oder daraus besteht, wobei das Material der äußeren Schicht (330) optional atmungsaktiv ist, wodurch es so konfiguriert ist, dass es Luft, nicht Flüssigkeit oder Körperflüssigkeiten, durchlässt.

12. Verfahren zur Herstellung eines urinabsorbierenden Beutels nach einem der Ansprüche 1 bis 11, umfassend die folgenden Schritte:
A. Herstellung der Innenschicht (300) durch eine Technik, die aus der Gruppe ausgewählt ist, die das Krempeln, die Spinnvliesherstellungstechniken, die Spunlace-Herstellungstechniken, die Laminat- und/oder gekoppelte und/oder perforierte Folienherstellungstechniken umfasst oder daraus besteht;
B. Herstellung der äußeren Schicht (330) durch ein Laminatmaterial;
C. Zusammenfügen der inneren Schicht (300), des absorbierenden Kerns und der äußeren Schicht (330) miteinander, um die ebene Mehrschichtstruktur (100) durch Verwendung chemischer Bindemittel und/oder mechanischer und/oder thermischer Techniken zu bilden;
D. Falten der ebenen Mehrschichtstruktur (100) entsprechend einer "C"-Form, dauerhaftes Schließen der Bodenkante (135) durch Schweißen und/oder Kleben, um den geschlossenen Boden (130) zu bilden, und dauerhaftes Verbinden des Endabschnitts (147) der zweiten Seitenkante (145) der ebenen Mehrschichtstruktur (100) mit dem ersten Seitenabschnitt (152) durch Schweißen und/oder Kleben.

13. Verfahren nach Anspruch 12, wobei der urinabsorbierende Beutel der absorbierende Beutel nach Anspruch 4 oder 5 oder nach einem der Ansprüche 6 bis 11 ist, wenn er von Anspruch 4 abhängt, ferner umfassend den folgenden Schritt vor Schritt C:
E1. Herstellen des absorbierenden Kerns in einer Linie (auf einer Linie) durch Dosieren des mindestens einen absorbierenden Pulvers in den Absorptionsbereich (325) mit variierenden Gewichten, wahlweise in kontinuierlicher und/oder abgestufter Weise, entlang der Längsachse (110) und/oder entlang einer Achse orthogonal zur Längsachse (110), wobei das mindestens eine absorbierende Pulver dann zwischen zwei Schichten aus Zellulosematerial eingeschlossen und zwischen ihnen durch Heißschmelzklebstoffe vereinigt und gehalten wird; oder
E2. die Herstellung des absorbierenden Kerns durch Einbringen von absorbierendem Material, das das mindestens eine absorbierende Pulver enthält, außerhalb der Linie.

14. Verfahren nach Anspruch 13, wobei der urinabsorbierende Beutel der absorbierende Beutel nach einem der Ansprüche 7 oder 8 oder nach einem der Ansprüche 9 bis 11 ist, wenn er von Anspruch 7 abhängt, ferner umfassend den folgenden Schritt vor Schritt C:
F. Herstellen der Zwischenverteilungsschicht (310) durch eine Technik, die aus der Gruppe ausgewählt ist, die Krempeln, Spinnvliesherstellungstechniken, Spunlace-Herstellungstechniken, Laminat- und/oder gekoppelte und/oder perforierte Blattherstellungstechniken umfasst oder daraus besteht;
wobei in Schritt C auch die Zwischenverteilungsschicht (310) mit der Innenschicht (300), dem absorbierenden Kern und der Außenschicht (330) zusammengefügt wird.

15. Verfahren nach Anspruch 14, wobei der urinabsorbierende Beutel der absorbierende Beutel nach Anspruch 9 oder nach Anspruch 10 oder 11 ist, wenn er von Anspruch 9 abhängt, ferner umfassend den folgenden Schritt nach Schritt C:
G. Ausführen eines Musters und/oder einer Prägung auf der inneren Schicht (300) in Übereinstimmung mit dem Absorptionsbereich (325), um das eine oder die mehreren ersten Übertragungsmuster und/oder Prägelinien (500) und/oder das eine oder die mehreren zweiten Sperrmuster und/oder Prägelinien zu erhalten.

## Revendications

1. Sac absorbant l'urine conçu pour loger le pénis d'un utilisateur, ayant une structure multicouche plane (100) pliée en forme de "C" pour former ledit sac absorbant présentant un axe longitudinal (110), où ledit sac absorbant a une ouverture supérieure (120), conçue pour recevoir le pénis de l'utilisateur, qui est délimitée par un bord supérieur (125) de la structure multicouche plane (100), où ledit sac absorbant comprend en outre un fond fermé (130) défini par un bord inférieur (135) de la structure multicouche plane (100), une première partie latérale (151) de la structure multicouche plane (100) chevauchant une partie centrale (150) de la structure multicouche plane (100) de sorte qu'un premier bord latéral (140) de celle-ci est placé sous une deuxième partie latérale (152) de la structure multicouche plane (100), où la structure multicouche plane (100) comprend une couche interne (300) conçue pour entrer en contact avec le pénis de l'utilisateur, une couche externe (330) et une âme absorbante interposée entre la couche interne (300) et la couche externe (330), où l'âme absorbante est conçue pour absorber et retenir un fluide, où une partie d'extrémité (147) d'un deuxième bord latéral (145) de la structure multicouche plane (100), s'étendant longitudinalement à partir du bord inférieur (135) sur une longueur non supérieure à 50 % de la longueur du deuxième bord latéral (145), est couplée de manière permanente sur la première partie latérale (151).

2. Sac absorbant selon la revendication 1, où la partie d'extrémité (147) du deuxième bord latéral (145) de la structure multicouche plane (100) s'étend longitudinalement depuis le bord inférieur (135) sur une longueur non supérieure à 40 % de la longueur du deuxième bord latéral (145).

3. Sac absorbant selon la revendication 1 ou 2, où la couche interne (300) ou la couche externe (330) est pourvue d'une étiquette adhésive (340 ; 340' ; 340") conçue pour coupler de manière réglable le deuxième bord latéral (152) sur la première partie latérale (151), l'étiquette adhésive (340 ; 340' ; 340") étant optionnellement repositionnable et/ou extensible.

4. Sac absorbant selon l'une quelconque des revendications précédentes, où l'âme absorbante comprend une zone d'absorption de liquide et de fluide (325) constituée d'au moins une poudre absorbante, optionnellement une poudre de polymère super absorbant ou SAP, piégée entre deux films de matière cellulosique, une quantité de ladite au moins une poudre absorbante dans la zone d'absorption (325) variant, optionnellement de manière continue et/ou étagée :
- selon l'axe longitudinal (110), la zone d'absorption (325) étant divisée en deux zones, optionnellement d'égale extension dans le plan de la structure multicouche plane (100), consistant en une zone (325d ; 325d') à absorption réduite et une zone de stockage (325e ; 325e'), où la zone (325d ; 325d') à absorption réduite est plus proche du bord supérieur (125) par rapport à la zone de stockage (325e ; 325e'), où la quantité de ladite au moins une poudre absorbante est supérieure dans la zone de stockage (325e ; 325e') par rapport à la zone (325d ; 325d') à absorption réduite ; et/ou
- selon un axe orthogonal à l'axe longitudinal (110).

5. Sac absorbant selon la revendication 4, où la quantité de ladite au moins une poudre absorbante dans la zone d'absorption (325) varie le long de l'axe longitudinal (110) et la quantité de ladite au moins une poudre absorbante dans la zone de stockage (325e ; 325e') varie de 60% à 90%, optionnellement est égale à au moins 70%, de la quantité de ladite au moins une poudre absorbante dans toute la zone d'absorption (325).

6. Sac absorbant selon l'une quelconque des revendications précédentes, où la zone d'absorption (325) est composée d'au moins trois sous-zones d'absorption (325a, 325b, 325c) s'étendant parallèlement à l'axe longitudinal (110), où au moins deux canaux d'écoulement longitudinaux (327b, 327c) dépourvus de ladite au moins une poudre absorbante séparent lesdites au moins trois sous-zones d'absorption (325a, 325b, 325c) les unes des autres.

7. Sac absorbant selon l'une quelconque des revendications précédentes, où la structure multicouche plane (100) comprend en outre une couche de distribution intermédiaire (310) interposée entre la couche interne (300) et l'âme absorbante, où la couche de distribution intermédiaire (310) est conçue pour recueillir et distribuer des fluides corporels vers la zone d'absorption (325).

8. Sac absorbant selon la revendication 7, où la couche de distribution intermédiaire (310) est réalisée en un matériau composé :
- de fibres naturelles, optionnellement choisies dans le groupe comprenant ou constitué de celluloses, de dérivés de cellulose, de viscose, de bambou, et de combinaisons de ceux-ci, et/ou
- de fibres polymères synthétiques, optionnellement choisies dans le groupe comprenant ou constitué de polyéthylène, de polypropylène, de polyester, de fibres à deux composants, et de combinaisons de ceux-ci, et/ou
- de combinaisons desdites fibres naturelles et desdites fibres polymères synthétiques.

9. Sac absorbant selon l'une quelconque des revendications précédentes, où la couche interne (300) est pourvue :
- d'une ou plusieurs, optionnellement deux, premières lignes de transfert à motif et/ou à gaufrage (500) s'étendant le long d'une direction parallèle à l'axe longitudinal (110) sur une longueur longitudinale optionnellement non inférieure à 60 % d'une longueur longitudinale de la zone d'absorption (325), où lesdites une ou plusieurs premières lignes de transfert à motif et/ou à gaufrage (500) sont conçues pour guider un fluide corporel vers le fond fermé (130), et/ou
- d'une ou plusieurs, optionnellement deux, deuxièmes lignes de barrière à motif et/ou à gaufrage (510) s'étendant selon une direction transversale à l'axe longitudinal (110), optionnellement en correspondance avec des bords transversaux de la zone d'absorption (325), optionnellement pour une longueur transversale non inférieure à 70 % de la longueur transversale de la zone d'absorption (325), où ladite ou lesdites deuxièmes lignes de barrière à motif et/ou à gaufrage (510) sont conçues pour fonctionner comme barrière de protection pour empêcher la fuite de fluides corporels stockés dans la zone d'absorption (325) à l'extérieur de celle-ci.

10. Sac absorbant selon l'une quelconque des revendications précédentes, où la couche interne (300) est constituée d'un matériau composé :
- de fibres naturelles, optionnellement choisies dans le groupe comprenant ou constitué de celluloses, de dérivés de cellulose, de coton, de bambou et de leurs combinaisons, et/ou
- de fibres polymères synthétiques, optionnellement choisies dans le groupe comprenant ou constitué de polyéthylène, de polypropylène, de fibres à deux composants, et de leurs combinaisons, et/ou
- de combinaisons desdites fibres naturelles et desdites fibres polymères synthétiques.

11. Sac absorbant selon l'une quelconque des revendications précédentes, où la couche externe (330) est constituée d'un matériau composite stratifié formé à partir d'un tissu non tissé avec un film de fibres polymères synthétiques, optionnellement choisi dans le groupe comprenant ou constitué de polyéthylène et/ou de polypropylène et/ou de combinaisons de ceux-ci, où le matériau de la couche externe (330) est optionnellement respirant, de sorte qu'il est conçu pour laisser passer l'air, et non les liquides ou les fluides corporels.

12. Procédé de fabrication d'un sac absorbant l'urine selon l'une quelconque des revendications 1 à 11 comprenant les étapes suivantes :
A. fabrication de la couche interne (300) par une technique choisie dans le groupe comprenant ou constitué du cardage, de techniques de fabrication de textile non-tissé filé-lié (spunbond), de techniques de fabrication de textile non-tissé lacé par filage (spunlace), de techniques de fabrication de feuilles stratifiées et/ou couplées et/ou perforées ;
B. fabrication de la couche externe (330) au moyen d'un matériau stratifié ;
C. assemblage de la couche interne (300), de l'âme absorbante et de la couche externe (330) les unes avec les autres pour former la structure multicouche plane (100) en utilisant des techniques à liants chimiques et/ou mécaniques et/ou thermiques ;
D. pliage de la structure multicouche plane (100) selon une forme en "C", fermeture de manière permanente du bord inférieur (135) par soudage et/ou adhésifs pour former le fond fermé (130), et couplage de manière permanente de la partie d'extrémité (147 ) du deuxième bord latéral (145) de la structure multicouche plane (100) sur la première partie latérale (152) par soudage et/ou adhésifs.

13. Procédé de fabrication selon la revendication 12, où le sac absorbant l'urine est le sac absorbant selon la revendication 4 ou 5, ou selon l'une quelconque des revendications 6 à 11, lorsqu'elle dépend de la revendication 4, comprenant en outre l'étape suivante avant l'étape C :
E1. réalisation en ligne (On Line) de l'âme absorbante par dosage de ladite au moins une poudre absorbante dans la zone d'absorption (325) avec des poids variables, optionnellement de manière continue et/ou étagée, selon l'axe longitudinal (110) et/ou selon un axe orthogonal à l'axe longitudinal (110), ladite au moins une poudre absorbante étant ensuite piégée entre deux couches de matériau cellulosique et réunies et maintenues entre elles par des adhésifs thermofusibles ; ou
E2. mise hors ligne de l'âme absorbante par introduction d'un matériau absorbant contenant ladite au moins une poudre absorbante.

14. Procédé de fabrication selon la revendication 13, où le sac absorbant l'urine est le sac absorbant selon l'une quelconque des revendications 7 ou 8, ou selon l'une quelconque des revendications 9 à 11, lorsqu'elles dépendent de la revendication 7, comprenant en outre l'étape suivante avant étape C :
F. fabrication de la couche de distribution intermédiaire (310) par une technique choisie dans le groupe comprenant ou constitué du cardage, de techniques de fabrication de textile non-tissé filé-lié (spunbond), de techniques de fabrication de textile non-tissé lacé par filage (spunlace), de techniques de fabrication de feuille stratifiée et/ou couplée et/ou perforée ;
où l'étape C assemble également la couche de distribution intermédiaire (310) avec la couche interne (300), l'âme absorbante et la couche externe (330).

15. Procédé de fabrication selon la revendication 14, où le sac absorbant l'urine est le sac absorbant selon la revendication 9 ou selon la revendication 10 ou 11, lorsqu'elle dépend de la revendication 9, comprenant en outre l'étape suivante après l'étape C :
G. réalisation d'un motif et/ou d'un gaufrage sur la couche interne (300) en correspondance avec la zone d'absorption (325) pour obtenir ledit ou lesdits premières lignes de transfert à motif et/ou à gaufrage (500) et/ou ladite ou lesdites deuxièmes lignes de barrière à motif et/ou à gaufrage.
